# EUROPEAN PATENT APPLICATION

(11) **EP 4 124 663 A1**
(43) Date of publication of application: **01.02.2023**
(21) Application number: 21306062.7
(22) Date of filing: 29.07.2021
(51) Int. Cl.: C12Q 1/6886

(54) **METHODS FOR PREDICTING A RESPONSE TO CANCER TREATMENT**

(71) Applicant: Hastim, 31300 Toulouse (FR); Aresu, Luca, 10090 Buttigliera Alta (IT); Marconato, Laura, 40136 Bologna (IT)
(72) Inventor: ARESU, Luca, 10090 BUTTIGLIERA ALTA (IT)
(74) Representative: Plasseraud IP

(57) **Abstract**

The present invention pertains to methods for predicting the response of a patient to an anticancer agent such as a chemotherapeutic agent or an immunotherapeutic agent. The methods according to the invention comprise a step of detecting, in a biological sample obtained from a cancer patient, a mutation in *TP53* and/or *POT1* genes. The present inventor has indeed demonstrated that the presence of a mutation in *TP53* or *POT1* is associated with a poor response to immunotherapy or chemotherapy.

## Description

### Field of the Invention

The present invention pertains to methods for predicting the response of a patient to an anticancer agent based on the detection of mutations in *TP53* and/or *POT1* genes.

### Background of the Invention

Cancer encompasses a large family of diseases characterized by uncontrolled cell growth and division. It is a leading cause of death worldwide and accounted for nearly 10 million deaths in 2020 (Ferlay et al. Global Cancer Observatory: Cancer Today. Lyon: International Agency for Research on Cancer; 2020). Although significant improvements have been made in diagnosis, screening and treatment, cancer still inflicts a terrible social burden in terms of loss of life, diminished quality of life, healthcare costs and reduced productivity.

Many types of cancer treatments have been developed and are available such as chemotherapy, radiotherapy, surgery and immunotherapy. Immunotherapy is now a recognized and well-established therapeutic strategy in the fight against cancer. It aims at instructing or reactivating the immune system to recognize cancer cells as foreign, ultimately eliminating them. The cells of the immune system are normally capable of monitoring and detecting the presence of abnormalities within the cells of the body. However, most tumors develop several mechanisms in order to escape this monitoring, leading to tolerance of the immune system toward the tumor. Stimulating the immune cells, such as T lymphocytes, via immunotherapy so that they specifically recognize the tumor cells makes it possible to efficiently overcome this tolerance. This stimulation can, for example, be carried out by directly bringing tumor antigens into contact (*in vitro* or *in vivo*) with antigen-presenting cells (APCs), thereby stimulating T lymphocytes. This is the concept of a therapeutic cancer vaccine. For an *in vivo* stimulation, the principle is to isolate abnormal proteins from the tumor and to reinject them in the patient in a form that is visible to the immune system.

Promising cancer vaccines based on the use of hydroxyapatite (HAP) particles have been developed. HAP can advantageously be used for purifying vaccinating proteins from tissue extracts or directly from blood samples and, once the HAP powder has adsorbed the tumor-specific tumor antigens, be directly administered as a cancer vaccine (see e.g. patent applications published under references WO 2006/122914, WO 2014/184453 or WO2019/175500). HAP powder is brought into contact with tumor proteins in order to produce a therapeutic antitumor vaccine which increases the level of immunity without any toxicity or side effect. This technique is safe, can be combined with chemotherapy and produces no toxic residue. This type of immunotherapy can make the difference between short- and long-term remission in certain cancers with a poor prognosis. This has for example been demonstrated in veterinary medicine, where this vaccination increases the survival of dogs with a deadly cancer such as diffuse large B-cell lymphoma (DLBCL) compared to chemotherapy alone. Excellent results have also been obtained for solid tumors such as bone cancers (osteosarcomas), mastocytomas or melanomas.

Unfortunately, some patients do not respond to first-line anticancer treatments such as immunotherapy or chemotherapy. Finding therapeutic alternatives for these patients is difficult and the efficiency of such treatments is generally hindered by the delay resulting from the elaboration of a new therapeutic strategy.

Thus, there is a continuous need for new biomarkers allowing for an accurate evaluation of the likeliness of a patient to respond to a known treatment, in particular to first-line treatments such as immunotherapy and chemotherapy.

### Summary of the Invention

The invention is defined by the claims.

Using a dog model, the present inventor has demonstrated that the presence of mutations in the *TP53* and the *POT1* genes is highly predictive of a patient's ability to respond to anticancer treatments such as chemotherapy and immunotherapy.

The present invention thus pertains to a method for predicting the response of a patient suffering from cancer to an anticancer agent, said method comprising a step of detecting, in a biological sample obtained from said patient, a mutation in the *TP53* and/or *POT1* genes. The invention also relates to an anticancer agent for use in the treatment of cancer in a patient, wherein said patient has been selected as not displaying a mutation in the *TP53* and *POT1* genes.

The anticancer agent is immunotherapy and/or chemotherapy. A preferred immunotherapy is an autologous anticancer vaccine such as that described in patent applications published under references WO 2014/184453 and WO2019/175500, i.e. comprising HAP powders loaded with tumor antigens.

### Detailed Description

Using a canine model of diffuse large B-cell lymphoma (DLBCL), the present inventor has demonstrated that mutations in *TP53* and/or in *POT1* are associated with a shorter survival (overall survival or progression-free survival) in patients either treated with chemotherapy or with a combination of chemotherapy and immunotherapy.

Dogs are considered as an excellent model for human cancer research: they spontaneously develop the same cancers as humans and have high similarities in terms of histological appearance, tumor genetics, physio-pathological pathways and treatment response (see e.g. Rowell et al. Trends in molecular medicine vol. 17,7 (2011): 380-388). The canine immune system is physiologically similar to that of humans and more closely related to humans than the immune system of mice. Cancer treatments used in dogs are similar to those used in humans (such as the "CHOP" chemotherapy which comprises a combination of cyclophosphamide, doxorubicin, vincristine and prednisone), and spontaneous cancers in dogs favored the development of several human-applied therapies such as sunitinib, a cKit inhibitor which is used for the treatment of renal cell carcinoma and gastrointestinal stromal tumors, or ganetespib, a heat shock protein 90 (HSP90) inhibitor which exhibits potent cytotoxicity in a wide variety of hematological and solid tumor cell lines (Mestrinho, L. A. & Santos, R. R. Advanced Drug Delivery Reviews 172, (2021): 296-313).

The data provided therein thus demonstrate that *TP53* and *POT1* can be efficiently used as biomarkers for predicting the response to anticancer agents in dogs, but also in other animals or humans. The present inventor has thus shown that the detection of mutations in the *TP53* and/or in the *POT1* genes allows predicting whether a patient will successfully respond to an anticancer agent such as chemo and/or immunotherapy.

Accordingly, the present invention relates to a method for predicting the response of a patient suffering from cancer to an anticancer agent, said method comprising a step of detecting, in a biological sample obtained from said patient, a mutation in *TP53* and/or *POT1* genes.

In the context of the present invention, the presence of a mutation in *TP53* and/or *POT1* is indicative of a reduced ability to respond to an anticancer agent, i.e. is indicative of a non- or poor- responder to the anticancer agent. Alternatively, in the context of the present invention, patients who do not display any mutation in *TP53* and *POT1* will be more likely to respond to a treatment with an anticancer agent, i.e. be a responder to the anticancer agent.

***"TP53"*** or **"Tumor Protein p53"** gene is a gene which encodes a tumor suppressor protein containing transcriptional activation, DNA binding, and oligomerization domains. The encoded protein acts as a tumor suppressor in many tumor types by regulating expression of target genes, thereby inducing cell cycle arrest, apoptosis, senescence, DNA repair, or changes in metabolism. *TP53* mutations are associated with a variety of cancers. The sequence of *TP53* is well known by the skilled person and is available under the reference ENSG00000141510 in the Ensembl Gene Database or Gene ID: 7157 in the NCBI Entrez Gene databank.

*"**POT1**"* or **"protection of telomeres** 1" is a gene member of the telombin family and encodes a nuclear protein involved in telomere maintenance. This protein functions as a member of a multi-protein complex that binds to telomeres thereby regulating telomere length and protecting chromosome ends from illegitimate recombination, chromosome instability, and abnormal chromosome segregation. The sequence of *POT1* is well known by the skilled person and is available under the reference ENSG00000128513 in the Ensembl Gene Database or Gene ID: 25913 in the NCBI Entrez Gene databank.

A **"mutation"** refers to any detectable change in genetic material, e.g. DNA, RNA, cDNA, or in an amino acid sequence encoded by such a genetic material. This includes gene mutations, in which the structure (e.g. DNA sequence) of a gene is altered, as well as protein mutations, in which the amino-acid structure of the protein is altered. Generally, a mutation is identified in a subject by comparing the sequence of a nucleic acid or of a polypeptide expressed in said subject with the corresponding nucleic acid or polypeptide expressed in a control population. When the mutation to be detected is a somatic mutation, i.e. a mutation that occurs in a specific cell line other than the germline, it can also be detected by comparing the sequence of a nucleic acid or of a polypeptide expressed by said cell line (e.g. tumor cells) with the corresponding nucleic acid or polypeptide expressed by another cell line (e.g. a non-cancerous cell line).

In the context of the present invention, the mutation in *TP53* or *POT1* refers to a mutation of one or more nucleotides in the *TP53* or *POT1* gene/mRNA sequence. Such a mutation can be a deletion (i.e. removal of at least one nucleotide in the gene sequence), an insertion (i.e. addition of at least one nucleotide in the gene sequence) or a substitution (i.e. replacement of one nucleotide with another). In the context of the present invention, the mutation is preferably located in the exons (i.e. in the coding parts of the gene). Mutations can also occur in the introns (i.e. the noncoding regions of the gene). Mutations can have an effect on the protein encoded by the gene such as missense mutations (which result in a codon that codes for a different amino acid), nonsense mutations (which result in a premature stop codon or a nonsense codon in the transcribed mRNA and thus in a truncated, and usually non-functional protein) or frameshift mutations (which result from the insertion or deletion of a number of nucleotides in a DNA sequence that is not divisible by three and induce a change the reading frame resulting in a completely different translation from the original). Mutations can also have no effect on the protein sequence (silent mutations).

According to a preferred embodiment, the mutation of *TP53* or *POT1* detected in the context of the present invention is a missense mutation.

A person skilled in the art knows many techniques allowing to detect a mutation within a gene.

The presence of a mutation in *TP53* and *POT1* genes according to the present invention may be detected by analysing *TP53* and *POT1* nucleic acid molecules. In the context of the invention, nucleic acid molecules include mRNA, genomic DNA and cDNA derived from mRNA. DNA or RNA can be single stranded or double stranded. These may be utilized for detection by amplification and/or hybridization with a probe, for instance. The nucleotide sequence can be obtained from a genomic DNA sample isolated from the biological sample. In this case, any biological sample containing genomic DNA can be used.

*TP53* and *POT1* mutations may be detected in RNA or DNA samples, preferably after amplification. For instance, the isolated RNA may be subjected to coupled reverse transcription and amplification, such as reverse transcription and amplification by polymerase chain reaction (RT-PCR), using specific oligonucleotide primers that are specific for the mutated site or that enable amplification of a the region containing the mutated site. Conditions for primer annealing may be chosen to ensure specific reverse transcription (where appropriate) and amplification; so that the appearance of an amplification product be a diagnostic of the presence of a mutation according to the invention. Otherwise, RNA may be reverse-transcribed and amplified, or DNA may be amplified, after which the mutated site may be detected in the amplified sequence by hybridization with a suitable probe or by direct sequencing, or any other appropriate method known in the art. For instance, a cDNA obtained from RNA may be cloned and sequenced to identify the mutated sequence of *TP53* and *POT1.*

As discussed above, the mutation detected in the *TP53* and *POT1* genes can result in mutated mature TP53 and POT1 proteins. Thus, the mutation of *TP53* or *POT1* may be detected at the protein level by detecting the mutated form of the TP53 and POT1 proteins. In this case, any biological sample wherein the mature protein is present may be used for detecting mutated forms of this protein. In this context, a sample, such as a tissue biopsy, obtained from a patient may be analysed in presence of antibodies specific of the mutated form or the protein, i.e. antibodies that are capable of distinguishing between the mutated form and the wild-type protein (or any other protein), to determine the presence or absence of the mutation specified by the antibody.

The antibodies may be monoclonal or polyclonal antibodies, single chain or double chain, chimeric antibodies, humanized antibodies, or portions of an immunoglobulin molecule, including those portions known in the art as antigen binding fragments Fab, Fab', F(ab')2 and F(v). They can also be immunoconjugated, e.g. with a toxin, or labelled antibodies.

Whereas polyclonal antibodies may be used, monoclonal antibodies are preferred because they are more reproducible in the long run.

Polyclonal antibodies can be obtained from serum of an animal immunized against the appropriate antigen, which may be produced by genetic engineering for example according to standard methods well-known by one skilled in the art (see e.g. Harlow et al. (1988)).

Monoclonal antibodies refer to a population of antibody molecules that contains only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Laboratory methods for preparing monoclonal antibodies are well known in the art (see, for example, Harlow et al., 1988).

Aptamers, which are a class of molecule that represents an alternative to antibodies in term of molecular recognition, can also be used for detecting the mutated form of *TP53* or *POT1* in the context of the present invention. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).

All the probes, primers, aptamers or antibodies used in the context of the present invention may be labelled with a detectable molecule or substance, such as a fluorescent molecule, a radioactive molecule or any others labels known in the art. Labels are known in the art that generally provide (either directly or indirectly) a signal.

In the context of the present invention, the "biological sample" can be any sample allowing for the detection of a mutation in *TP53* or *POT1. TP53* and *POT1* mutations according to the present invention are advantageously detected in the tumor genome, i.e. in genetic material derived from tumor cells. The mutations detected in the context of the present invention are thus preferably somatic mutations displayed by the tumor cells, i.e. mutations that occur only in cancer cells. Such mutations can thus be detected by comparing the sequence of TP53 or POT1 (genes or protein) expressed in cancer cells with that of TP53 or POT1 expressed in other cell lines, in particular cells derived from healthy tissues. The biological sample according to the present invention is thus a sample comprising tumor-derived genetic material and allowing for the detection of mutations comprised in the tumor genome. Examples of such samples include fluids, tissues, cell samples, organs, biopsies, etc. Preferred biological samples are a cell or tissue sample. The biological sample can be whole blood, serum or plasma obtained from the patient. In a preferred embodiment according to the present invention, the biological sample is a tumor sample obtained from the patient.

The method according to the present invention allows predicting a patient's response to a treatment with an anticancer agent, i.e. whether the patient will benefit (respond) or not from receiving the treatment. Patients who will benefit from receiving the treatment are also referred to as **"responders".** Administering the treatment to these patients will allow treating their disease and/or improving their survival (either overall survival or disease-free survival). Responders will achieve a response, i.e. the cancer is eradicated, reduced or stabilized after treatment, and show a longer survival time as compared to non-responders. On the contrary, patients who will not benefit from receiving a known treatment are referred to **"non-responders"** or **"poor-responders".** Administering the treatment to these patients will not allow treating their disease or at least less efficiently than in responders, and/or the treatment will not significantly improve their survival (either overall survival or disease-free survival). Non-responders and poor-responders have a shorter survival time than responders after treatment. For these patients, the selection of an alternative treatment strategy would thus be advantageous.

The method according to the present invention allows for the correct and efficient identification of responders to anticancer agents. Such patients do not display a mutation in the *TP53* and/or *POT1* genes.

Accordingly, in a further embodiment, the present invention pertains to an anticancer agent for use in the treatment of cancer in a patient, wherein said patient has been selected as not displaying a mutation in the *TP53* and *POT1* genes

The present disclosure also provides a method for treating a patient suffering from cancer comprising administering to said patient a therapeutically effective amount of an anticancer agent, wherein said patient has been selected as not displaying a mutation in the *TP53* and *POT1* genes.

By a **"therapeutically effective amount"** is meant a sufficient amount to treat cancer.

The **"patient"** or **"subject"** is a human being or an animal, for example a mammal, and in particular dogs, horses or cats.

The **"cancer"** according to the present invention can be of various origin (such as breast, colon, rectum, lung, head and neck, bladder, ovary, prostate), and involve various cancer cell types (adenocarcinoma, squamous cell carcinoma, large cell cancer, melanoma, etc).

In one particular embodiment, the cancer is chosen from the group consisting of lymphomas, osteosarcomas, mammary tumors, melanomas, hemangiosarcomas, mast cell tumors, fibrosarcomas, brain or central nervous system tumors, schwannomas, mesotheliomas, seminomas, teratomas and blastomas. The cancer can particularly be chosen from a glioblastoma, a sarcoma (such as osteosarcoma or fibrosarcoma), a melanoma, a carcinoma or an adenocarcinoma.

According to a preferred embodiment, the cancer is a lymphoma (either a non-Hodgkin lymphoma or a Hodgkin lymphoma). According to a more preferred embodiment, said cancer is a non-Hodgkin lymphoma such as diffuse large B-cell lymphoma (DLBCL).

The cancer can be a disseminated cancer, that is to say one which has metastases (patients classified in the NxMx category according to the TNM classification).

An anti-cancer treatment generally consists of radiotherapy, surgery, chemotherapy (with a chemotherapeutic agent) or immunotherapy (with an immunotherapeutic agent). The treatment may consist of an adjuvant therapy (i.e. treatment after surgical resection of the primary tumor) or of a neoadjuvant therapy (i.e. treatment before surgical resection of the primary tumor).

As shown in the examples below, the method according to the present invention particularly allows predicting the response of a patient suffering from cancer to chemotherapy, immunotherapy or a combination thereof. Thus, according to a preferred embodiment, the "anticancer agent" according to the present invention is an immunotherapeutic agent, a chemotherapeutic agent or a combination of an immunotherapeutic agent with a chemotherapeutic agent.

**"Immunotherapy"** aims at activating the patient's own immune system to fight against the disease. An **"immunotherapeutic agent"** refers to a compound, composition or treatment that indirectly or directly enhances, stimulates or increases the body's immune response against cancer cells. Cancer immunotherapy agents include nucleic acids, cytokines, peptides, proteins, immune cells (endogenous, or conferred with anti-cancer activity ex vivo), fragments of bacteria or viruses, and synthetic drugs. They can be used to elicit a specific immune response against a particular cancer cell type, or to trigger a general immune response that indirectly targets cancer cells or their effects. The former is typically achieved with antibodies or vaccines that target one or more antigens on or in cancer cells. Immunotherapy is usually done with immunomodulatory agents and/or chemical entities that simultaneously activate one or more types of immune cells to fight against cancer cells.

Immunotherapeutic agents can be non-specific, i.e. boost the immune system generally so that the human body becomes more effective in fighting the growth and/or spread of cancer cells, or they can be specific, i.e. targeted to the cancer cells themselves. Immunotherapy regimens may combine the use of non-specific and specific immunotherapeutic agents.

Non-specific immunotherapeutic agents are substances that stimulate or indirectly improve the immune system. Non-specific immunotherapeutic agents have been used alone as a main therapy for the treatment of cancer, as well as in addition to a main therapy, in which case the non-specific immunotherapeutic agent functions as an adjuvant to enhance the effectiveness of other therapies (e.g. cancer vaccines). Non-specific immunotherapeutic agents can also function in this latter context to reduce the side effects of other therapies, for example, bone marrow suppression induced by certain chemotherapeutic agents. Non-specific immunotherapeutic agents can act on key immune system cells and cause secondary responses, such as increased production of cytokines and immunoglobulins. Alternatively, the agents can themselves comprise cytokines. Non-specific immunotherapeutic agents are generally classified as cytokines or non-cytokine adjuvants.

A number of cytokines have found application in the treatment of cancer either as general non-specific immunotherapies designed to boost the immune system, or as adjuvants provided with other therapies. Suitable cytokines include, but are not limited to, interferons, interleukins and colony-stimulating factors.

Interferons (IFNs) include the common types of IFNs, IFN-alpha (IFN-a), IFN-beta (IFN-beta) and IFN-gamma (IFN-y). IFNs can act directly on cancer cells, for example, by slowing their growth, promoting their development into cells with more normal behavior and/or increasing their production of antigens thus making the cancer cells easier for the immune system to be recognized and destroyed. IFNs can also act indirectly on cancer cells, for example, by slowing down angiogenesis, boosting the immune system and/or stimulating natural killer (NK) cells, T cells and macrophages. Recombinant IFN-alpha is available commercially as Roferon (Roche Pharmaceuticals) and Intron A (Schering Corporation). The use of IFN-alpha, alone or in combination with other immunotherapeutics or with chemotherapeutics, has shown efficacy in the treatment of various cancers such as melanoma (including metastatic melanoma), renal cancer (including metastatic renal cancer), breast cancer, prostate cancer, and cervical cancer (including metastatic cervical cancer).

Interleukins include e.g. IL-2and IL-11. Examples of commercially available recombinant interleukins include Proleukin^{®} (IL-2; Chiron Corporation) and Neumega^{®} (IL-12; Wyeth Pharmaceuticals). Zymogenetics, Inc. (Seattle, Wash.) is currently testing a recombinant form of IL-21, which is also contemplated for use in the combinations of the present invention. Interleukins, alone or in combination with other immunotherapeutics or with chemotherapeutics, have shown efficacy in the treatment of various cancers such as renal cancer (including metastatic renal cancer), melanoma (including metastatic melanoma), ovarian cancer (including recurrent ovarian cancer), cervical cancer (including metastatic cervical cancer), breast cancer, colorectal cancer, lung cancer, brain cancer, and prostate cancer.

Interleukins have also shown good activity in combination with IFN-alpha in the treatment of various cancers (Negrier et al., Ann Oncol. 2002 13(9): 1460-8 ; Touranietal, J. Clin. Oncol. 2003 21(21):398794).

Colony-stimulating factors (CSFs) include granulocyte colony stimulating factor (G-CSF or filgrastim), granulocyte-macrophage colony stimulating factor (GM-CSF or sargramostim) and erythropoietin (epoetin alfa, darbepoietin). Various-recombinant colony stimulating factors are available commercially, for example, Neupogen^{®} (G-CSF; Amgen), Neulasta (pelfilgrastim; Amgen), Leukine (GM-CSF; Berlex), Procrit (erythropoietin; Ortho Biotech), Epogen (erythropoietin; Amgen), Arnesp (erytropoietin). Colony stimulating factors have shown efficacy in the treatment of cancer, including melanoma, colorectal cancer (including metastatic colorectal cancer), and lung cancer.

Non-cytokine adjuvants include Levamisole, alum hydroxide (alum), Calmette-Guerin bacillus (ACG), incomplete Freund's Adjuvant (IFA), QS-21, DETOX, Keyhole limpet hemocyanin (KLH) and dinitrophenyl (DNP). Non-cytokine adjuvants in combination with other immuno- and/or chemotherapeutics have demonstrated efficacy against various cancers including, for example, colon cancer and colorectal cancer (Levimasole); melanoma (BCG and QS-21); renal cancer and bladder cancer (BCG).

In addition to having specific or non-specific targets, immunotherapeutic agents can be active, i.e. stimulate the body's own immune response (such as cancer vaccines), or they can be passive, i.e. comprise immune system components that were generated external to the body.

The immunotherapeutic treatment may consist of an adoptive immunotherapy as described by Nicholas P. Restifo, Mark E. Dudley and Steven A. Rosenberg "Adoptive immunotherapy for cancer: harnessing the T cell response, Nature Reviews Immunology, Volume 12, April 2012). In adoptive immunotherapy, the patient's circulating lymphocytes, or tumor infiltrated lymphocytes, are isolated in vitro, activated by lymphokines such as IL-2 or transuded with genes for tumor necrosis, and readministered (Rosenberg et al., 1988; 1989). The activated lymphocytes are most preferably the patient's own cells that were earlier isolated from a blood or tumor sample and activated (or "expanded") in vitro. This form of immunotherapy has produced several cases of regression of melanoma and renal carcinoma in human patients.

Immune checkpoint inhibitor or checkpoint blockade cancer immunotherapy agent can also be used as immunotherapeutic agents. These compounds inhibit the function of an immune inhibitory checkpoint protein. Inhibition includes reduction of function and full blockade. Immune checkpoint inhibitors include peptides, antibodies, nucleic acid molecules and small molecules. Preferred immune checkpoint inhibitors are antibodies that specifically recognize immune checkpoint proteins. Such antibodies can e.g. be selected from the group consisting of anti-PD1 antibodies (such as e.g. nivolumab or lambrolizumab), anti-PDL1 antibodies (such as e.g. Atezolizumab, Avelumab or Durvalumab), anti-PDL2 antibodies, anti-CTLA4 antibodies (such as tremelimumab or ipilimumab), anti-TIM-3 antibodies, anti-LAG3 antibodies, anti-IDO1 antibodies, anti-TIGIT antibodies, anti-B7H3 antibodies, anti-B7H4 antibodies, anti-BTLA antibodies and anti-B7H6 antibodies.

Cancer vaccines are preferred immunotherapeutic agents in the context of the present invention. The goal of therapeutic cancer vaccines is to induce tumour regression, eradicate minimal residual disease, establish lasting antitumour memory and avoid non-specific or adverse reactions (Saxena, Mansi, et al. Nature Reviews Cancer 21.6 (2021): 360-378). Cancer vaccines particularly aim at instructing the cancer patient's own immune system to directly attack and destroy cancer cells. They thus exert an antitumor effect by engaging the host immune response, particularly T cells, and have the great potential of circumventing the intrinsic drug resistance that leads to chemotherapy failure. Tumor vaccines generally involve two classes of proteins: tumor-associated antigens (TAAs) and tumor-specific antigens (TSAs). TAAs are self-antigens that may be expressed in normal cells but that are either preferentially or abnormally expressed in tumor cells. TSAs encompass antigens expressed by oncoviruses and neoantigens encoded by cancer mutations, and are truly tumor-specific (see for review Hollingsworth, Robert E., and Kathrin Jansen. npj Vaccines 4.1 (2019): 1-10; Esfahani, K., et al. Current Oncology 27.s2 (2020): 87-97; or Emens, Leisha A. Expert opinion on emerging drugs 13.2 (2008): 295-308). Examples of cancer vaccines that have be approved for the treatment of various cancers include cancer vaccine HSPPC-96 or Sipuleucel-t.

According to a preferred embodiment, the cancer vaccine used in the context of the present invention is an autologous cancer vaccine. An **"autologous"** vaccine denotes a vaccine in which the tumor proteins/antigens are obtained from the patient intended to be vaccinated. Such vaccines are typically produced by obtaining tumor cells/tumors antigens from the patient and manipulating them to make them more recognizable by the patient's immune system. The treated tumor cells/tumors antigens are then injected into the patient's body to elicit an immune response directed against this population of cells/antigens, i.e. against the tumor. Ideally, memory immune cells persist in the body and allow targeting new cancer cells, should the cancer return.

According to a preferred embodiment, the cancer vaccine according to the present invention is an autologous vaccine comprising particles of hydroxyapatite and/or of tricalcium phosphate loaded with tumor antigens such as those disclosed in patent applications published under references WO 2014/184453 or WO2019/175500 (such as the Apavac^{®} vaccine) whose contents are incorporated herein by way of reference.

The notion of "tumor proteins/antigens" is well known to those skilled in the art. They are proteins and/or molecules expressed specifically by the tumor cells and which can be recognized by T and B lymphocytes.

Tumor proteins/antigens can be extracted from tumor tissues (i.e. purified from tumor biopsies as shown in WO 2014/184453) or directly extracted from serum or plasma samples obtained from the cancer patient (as shown in WO2019/175500).

Typically, the weight of the tumor proteins/antigens extracted from the serum or plasma sample is between 60 kDa and 130 kDa, in particular between 70 kDa and 110 kDa. Those skilled in the art are aware of a large number of techniques for extracting proteins from a serum or plasma sample. Typically, the tumor proteins/antigens are extracted by precipitating the serum sample in a saline solution, the mixture thus obtained is then centrifuged, and the pellet obtained represents the extracted proteins.

The proteins/antigens extracted are brought into contact with the hydroxyapatite particles to produce an autologous vaccine comprising particles of hydroxyapatite and/or of tricalcium phosphate loaded with tumor antigens.

"Hydroxyapatite" is a mineral of the calcium phosphate family, of formula Ca₁₀(PO4)₆(OH)₂, and the unit cell of the crystalline structure is hexagonal. Hydroxyapatite is the hydroxylated member of the apatite group. The ions of the crystal unit cell can be replaced with other ions of similar charges and sizes; tunnels also exist in the unit cell which can receive small molecules such as certain amino acids. These are those particular features which give this mineral very specific adsorption properties.

The hydroxyapatite particles according to the present invention are obtained via the same method as that described in application WO 2014/184453. This document describes in particular a method for producing calcium phosphate hydroxyapatite Ca₁₀(PO4)₆(OH)₂ particles, consisting of a slow precipitation at high temperature, obtained by double decomposition of a calcium salt and of a phosphorus salt in a basic medium. The reaction is carried out at constant temperature in a large reaction volume and is followed by a maturation phase and a phase of washing with water. The precipitate thus obtained undergoes a further transformation step: solid/solution separation. The latter step is carried out either by filtration, drying by stoving and crushing, or by spray-drying using a fluidized bed. Regardless of the solid/solution separation technique chosen, the powder will undergo two transformation steps specific to the application of the invention: the step of particle size selection by dry screening so as to retain only the particle size band of interest, less than 25 µm or between 25 and 45 µm, then a final step during which the powder will be sintered at an optimal temperature to ensure fusion of the grains resulting in quite particular surface finishes of the powders (preferentially greater than or equal to 30 m²/g). These two final steps can be reversed: selection then sintering or sintering then selection. "Sintering" is a process consisting in heating a powder without bringing it to melting. In the context of the present invention, the sintering is for example carried out at a temperature between 400°C and 600°C. The HAP thus obtained may be in powder form and may undergo one or more washes.

Typically, the tumor proteins/antigens are brought into contact with hydroxyapatite particles by passing said tumor proteins/antigens over a column of hydroxyapatite particles, such as a chromatography column. The tumor antigens may optionally be brought into contact in solution and then washed by centrifugation. The tumor proteins/antigens are then adsorbed onto the surface of the hydroxyapatite particles. The hydroxyapatite particles may particularly be in powder form.

When a chromatography column is used, it can for example be placed under pressure.

Once the hydroxyapatite particles have been loaded with tumor proteins/antigens, they are suspended in an injection liquid and can be injected into the patient from whom the serum/plasma sample was obtained.

Typically, the injection liquid comprises an organic agent which facilitates the injection, such as carboxymethylcellulose.

The autologous vaccine comprising particles of hydroxyapatite and/or of tricalcium phosphate loaded with tumor antigens obtained as disclosed above is a preferred immunotherapeutic agent according to the present invention. Said autologous vaccine is typically obtained by:
a) extracting the proteins contained in a tumor, serum or plasma sample obtained from a patient suffering from cancer; and
b) bringing the proteins extracted in step a) into contact with particles of hydroxyapatite and/or of tricalcium phosphate in order to obtain the autologous vaccine.

Typically, the autologous vaccine is in the form of a suspension.

Typically, the weight of the tumor antigens extracted from the serum or plasma sample is between 60 kDa and 130 kDa, in particular between 70 kDa and 110 kDa.

This autologous vaccine is typically administered by injection, for example by subcutaneous or intradermal injection. The vaccine can be administered orally or by any other means allowing transmucosal vaccination.

The patient may receive one or more injections, doses of said autologous vaccine. A dose generally comprises between 30 and 50 mg of hydroxyapatite and/or of tricalcium phosphate and between 1000 and 2000 µg of proteins. The patient may receive several injections spaced out over time, for example several days, weeks or months. Injections are typically given on a weekly basis during the first month and then monthly for 4 months. Each injection can be ideally prepared from a new sample of tumor proteins from the patient in the event of escape from the vaccine or of inefficacy, in particular when there is an increase in the level of tumor markers.

The term **"chemotherapeutic agent"** refers to chemical compounds that are effective in inhibiting tumor growth. Examples of chemotherapeutic agents include cyclophosphamide; alkylating agents such as thiotepa and cyclophosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaorarnide and trimethylolomelamine; acetogenins (especially bullatacin and bullatacinone); a carnptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estrarnustine, ifosfamide, mechlorethamine, mechlorethamine oxide hydrochloride, melphalan, novembichin, phenesterine, prednimus tine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin (11 and calicheamicin 211, see, e.g., Agnew Chem Intl. Ed. Engl. 33:183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, canninomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idanrbicin, marcellomycin, mitomycins, mycophenolic acid, nogalarnycin, olivomycins, peplomycin, potfiromycin, puromycin, quelamycin, rodorubicin, streptomgrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophospharnide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pento statin; phenamet; pirarubicin; podophyllinic acid; 2-ethylhydrazide; procarbazine; PSK^{®}; razoxane; rhizoxin; sizofiran; spirogennanium; tenuazonic acid; triaziquone; 2,2',2"-trichlorotriethylarnine; trichothecenes (especially T-2 toxin, verracurin A, roridinA and anguidine); urethan; vindesine; dacarbazine; mannomustine; mitobromtol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, N.].) and doxetaxel (TAXOTERE^{®}, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-1 1 ; topoisomerase inhibitor RFS 2000; difluoromethylornithine (DMFO); retinoic acid; capecitabine; and pharmaceutically acceptable salts, acids or derivatives of any of the above. Also included in this definition are antihormonal agents that act to regulate or inhibit honnone action on tumors such as anti-estrogens including for example tamoxifen, raloxifene, aromatase inhibiting 4(5)-imidazoles, 4-mycinetamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

According to a preferred embodiment, the chemotherapeutic agent according to the present invention is a combination of cyclophosphamide, doxorubicin (such as hydroxydaunorubicin), vincristine (marketed under the brand name Oncovin^{®}) and a steroid selected from prednisone and prednisolone. This specific combination is also known as "CHOP" chemotherapy (acronym for "**C**yclophosphamide", "**H**ydroxydaunorubicin", "**O**ncovin^{®}" and "**P**rednisone").

The method according to the present invention thus specifically allows determining whether a patient suffering from cancer will respond to a chemotherapy consisting of a CHOP chemotherapy, to an immunotherapy consisting of an autologous cancer vaccine comprising particles of hydroxyapatite and/or tricalcium phosphate loaded with tumor antigens, or to a combination of these specific therapies.

The present invention is presented in greater detail in the examples below. These examples are provided only for illustrative purposes and cannot be interpreted as limiting the scope of the present invention.

### Brief Description of the Figures

**Figure 1****:** Dogs were categorized into 2 groups based on whether they had received chemotherapy (such as CHOP therapy) or a combination of chemotherapy (such as CHOP therapy) plus immunotherapy (such as Apavac^{®}). Curves of disease progression **(A)** and survival **(B)** over time are represented.
**Figure 2****:** Dogs treated with chemotherapy were categorized into 2 groups based on the presence of mutations in *TP53.* Curves of disease progression **(A)** and survival **(B)** over time are represented.
**Figure 3****:** Dogs treated with chemo-immunotherapy (CHOP + Apavac^{®}) were categorized into 2 groups based on the presence of mutations in *POT1* **(A)** or *TP53* **(B).** Curves of survival over time are represented.
**Figure 4****:** Clinical validation of *TP53* mutations by Sanger sequencing analysis: Dogs treated with chemo-immunotherapy (CHOP + Apavac^{®}) were categorized into 2 groups based on the presence of mutations in *TP53.* Curves of disease progression **(A)** and survival **(B)** over time are represented.

### EXAMPLES

### TP53 and POT1 Gene Mutations in canine diffuse large B-cell lymphoma: Cancer Risk and Genetic Testing

Lymphoma in domestic dogs is considered a representative and highly predictive spontaneous model for human disease. In particular, the complex genetics interplay, the intact immune system, the environmental exposures and the increasing incidence represent powerful elements for translational studies. Among the several lymphoma subtypes, canine diffuse large B-cell lymphoma (cDLBCL) is the most common, accounting for approximately 60-70% of hematological malignancies in this species. Current survival rates for cDLBCL after chemotherapy or immune-chemotherapy are usually disappointing and dogs show markedly different clinical courses and treatment responses, demonstrating a heterogeneous clinical behavior and a poor chance to anticipate the outcome. Proposed cDLBCL prognostic classification systems are based on bone marrow infiltration, substage, mitotic rate and histological features (centroblastic and immunoblastic) and do not take in consideration the mechanisms underlying the tumorigenesis. One large study based on whole exome sequencing (WES) investigated canine B-cell lymphomas obtained from three predisposed breeds (Boxer, Golden Retriever and Cocker Spaniel) and both TRAF3 and MAP3K14 were found frequently mutated. Notably, FBXW7 mutations were identified occurring in a specific codon recurrently mutated in several human cancers. Despite the large number of cases included in this study the mutations described may be breed-specific, thereby limiting the more heterogeneous mutational landscape of canine lymphoma. Furthermore, tumors were not appropriately classified by WHO criteria and clinico-pathological features were not reported. Thus, the clinical significance of these results remains unknown.

In the present study an integrated model including mutations, copy number aberrations was designed to predict overall survival and tumor free interval. Clinically significant mutations were further identified and clinically validated.

### Study population and clinical characteristics

Study was conducted using DNA from 77 de novo cDLBCL cases with matched normal tissues for WES. Briefly, mixed-breed dogs (19/77), German shepherds (9/77), Rottweilers (7/77) and Golden retrievers (4/77) were the most common breeds. There were 40 females and 37 males. At diagnosis, median age was 7 years (range 3-15 years) and median weight was 30.8 kg (range, 4.5-81.3 kg). Regarding clinical stage, 48 dogs had stage V disease, 28 had stage IV and only one dog had stage III disease. Forty-nine (63.6%) dogs were asymptomatic at presentation (substage a), while 28 (36.4%) showed symptoms (substage b). Overall, 22 dogs had bone marrow infiltration, with a median of 3% neoplastic cells (range, 1-50%). Peripheral blood infiltration was present in 43 dogs, with a median of 4% neoplastic cells (range, 1-74%). At presentation, 33 (42.9%) dogs had an increased level of serum LDH and 23 (29.9%) dogs had received steroids before being diagnosed with lymphoma.

Dogs received either chemotherapy (including CHOP therapy) alone or a combination of chemotherapy (CHOP therapy) plus immunotherapy (an autologous cancer vaccine: Apavac^{®}).

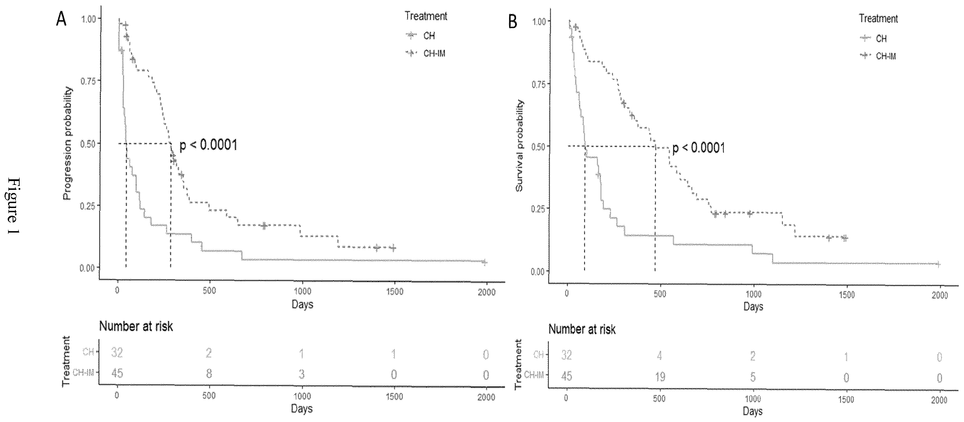

Treatment affected significantly both the time to progression (TTP) and the overall survival (LSS for "lymphoma-specific survival" - see **Figure 1****).** Indeed, dogs treated with chemo-immunotherapy (45/77) showed a favorable prognosis compared to dogs receiving chemotherapy alone (32/77) and also LSS in these dogs was significantly associated with substage and bone marrow infiltration. In dogs receiving chemotherapy only, peripheral blood infiltration correlated with both TTP and LSS, whereas bone marrow infiltration with LSS only *POT1* and *TP53* mutations in cDLBCL

Mutations were detected in tumor samples obtained from each dog included in the study.

Tumor genomic DNA (gDNA) was isolated to prepare libraries for WES. For each dog, gDNA from skin punch biopsies was also extracted and employed as matched-control for subsequent analyses. The exome was captured and then sequenced by Illumina NovaSeq (150 PE) with target coverage of 250×. Briefly, gDNA was tested for concentration and purity and then subjected to fragmentation. Illumina-compatible whole exome libraries were prepared using the SPRIworks HT Reagent Kit (Beckman Coulter, Inc., Indianapolis, Indiana, Cat. No. B06938) and the resulting libraries were subjected to exome enrichment using the SureSelectXT Canine All Exon Kit (Agilent, Cat. No. 5190-5452), following the manufacturer's instructions. Final concentration and size distribution were tested with Agilent Bioanalyzer 2100. Somatic mutations discovery across all samples was performed following the Genome Analysis Toolkit16 v.3.7 Best Practices (accessible via the Broad Institute website: Genome Analysis Toolkit/Getting Started/Best practices webpage). Single nucleotide variants (SNVs) and small insertion and deletions (indels) were identified with Mutect217 and Varscan18 v.2.4.1. Loss of heterozygosity (LOH) was further investigated by Varscan. SNVs, indels and LOH loci were considered as the union of Mutect2 and Varscan filtered somatic mutations.

We found several genes with mutations but *POT1* and *TP53* were two of most interesting. *POT1* mutations were identified in 28.6% of the cases. Missense mutations represented 54.5% of variants, while nonsense and frameshift mutations were 27.3% and 18.2%, respectively. A total of twenty-four mutations in *TP53* gene were reported in 19 dogs (24.7%). We identified 19 missense (79.2%), three frameshift (12.5%) and two nonsense variants (8.3%). Five dogs presented two *TP53* variants and one of them also carried a frameshift deletion and a nonsense variant on the same allele.

Purebred dogs were more frequently affected by *POT1* mutations compared to mixed breed dogs.

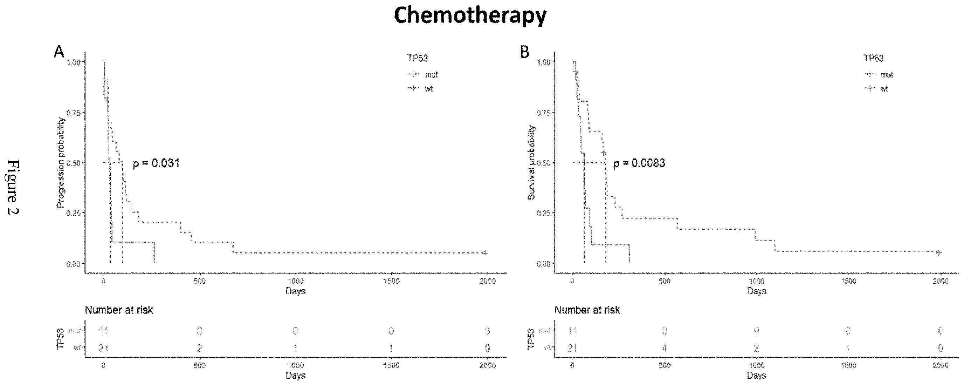

In dogs treated with chemotherapy alone *TP53* mutation was correlated with a shorter TTP and overall survival. The median TTP for *TP53* mutant and wt dogs was 32 and 98 days, respectively, while the median LSS for *TP53* mutant and wt dogs was 60 and 176 days, respectively (see **Figure 2****).**

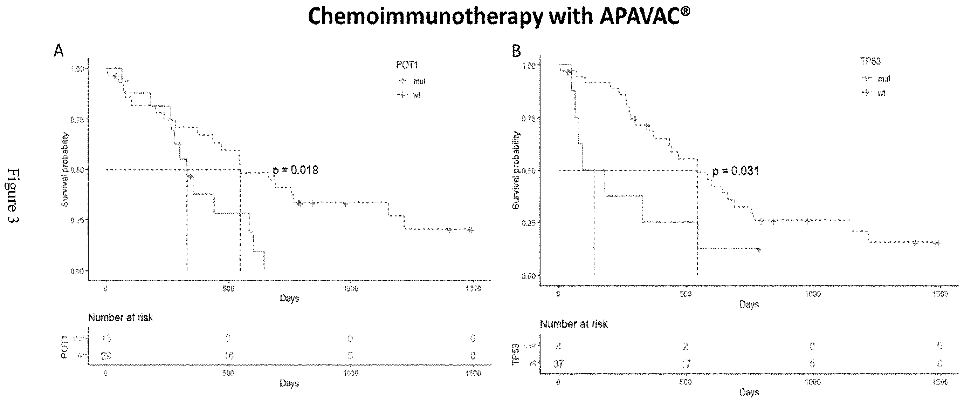

In dogs treated with chemo-immunotherapy *POT1* mutation was correlated with a shorter LSS. The median LSS for patients with mutant and wt *POT1* was 330 and 547 days, respectively (see **Figure 3A****).** Likewise, dogs carrying *TP53* mutation were associated with a shorter median LSS (see **Figure 3B****).** Also, mutations in both genes were not associated with any other clinical parameters.

The clinical validation of *TP53* mutations by Sanger sequencing analysis in cDLBCL treated with chemoimmunotherapy with APAVAC showed similar results to WES.

Briefly, Sanger sequencing was performed to validate the protein coding somatic mutations identified by WES in *TP53* gene. Primer pairs were specifically designed using Primer3 and Primer-BLAST tools. PCR was performed in a final volume of 20 µl using HotStarTaq DNA Polymerase kit (QIAGEN, Hilden, Germany) and 50 ng of gDNA with the following cycling conditions: initial denaturation at 95°C for 5 min, 33 cycles at 95°C for 30 s, at 58°C for 30 s and at 72°C for 40 s, with final extension at 72°C for 10 min. Exon 18 of *POT1* was amplified using Touchdown PCR as previously described (Nat. Protoc, 2008). Briefly, the touchdown phase was conducted with 20 cycles at annealing temperature of 65°C decreasing 0.5°C at each cycle, while the second phase was conducted with 25 cycles at annealing temperature of 55°C. PCR products were purified using the ExoSAP-IT PCR Product Cleanup Reagent (Applied Biosystems, Foster City, CA, USA). Purified products were sequenced in the forward or reverse direction using the BigDye Terminator v1.1 Cycle Sequencing Kit (Applied Biosystems) following manufacturer's instructions and analyzed on a SeqStudio Genetic Analyzer (Applied Biosystems). In total, 47 dogs were investigated for the set of mutations identified in the six exons.

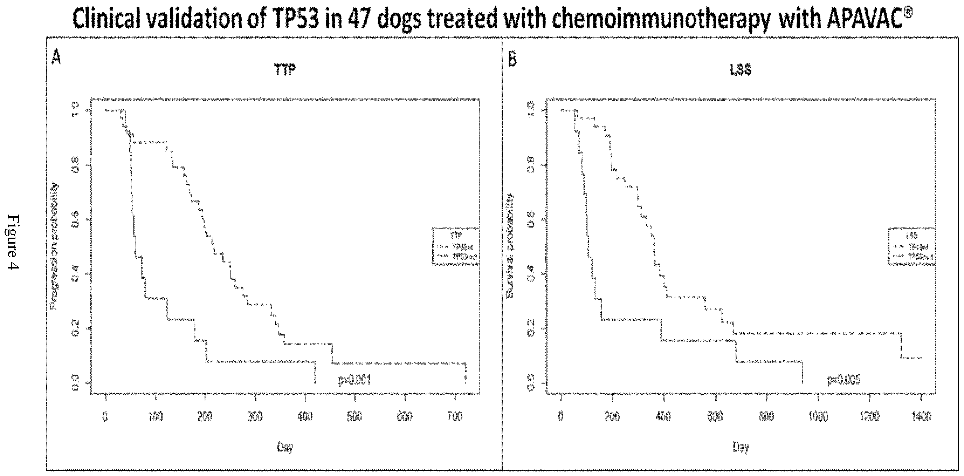

The median TTP for *TP53* mutant and wt dogs was 60 and 217 days, respectively (see **Figure 4A****),** while the median LSS for *TP53* mutant and wt dogs was 105 and 361 days, respectively (see **Figure 4B****).**

## Claims

1. A method for predicting the response of a patient suffering from cancer to an anticancer agent, said method comprising a step of detecting, in a biological sample obtained from said patient, a mutation in *TP53* and/or *POT1* genes.

2. The method according to claim 1, wherein the presence of a mutation in *TP53* and/or *POT1* genes is indicative of a non- or poor- responder to the anticancer agent.

3. The method according to claim 1 or 2, wherein said mutation is a missense mutation.

4. The method according to any one of claims 1 to 3, wherein said biological sample is a tumor sample.

5. The method according to claim 4, wherein said mutation is a somatic mutation displayed by tumor cells.

6. An anticancer agent for use in the treatment of cancer in a patient, wherein said patient has been selected as not displaying a mutation in *TP53* and *POT1* genes.

7. The method or the anticancer agent for use according to any one of claims 1 to 6, wherein said patient is a human.

8. The method or the anticancer agent for use according to any one of claims 1 to 6, wherein said patient is a dog, a horse or a cat.

9. The method or the anticancer agent for use according to any one of claims 1 to 8, wherein said cancer is chosen from the group consisting of lymphomas, osteosarcomas, mammary tumors, melanomas, hemangiosarcomas, mast cell tumors, fibrosarcomas, brain or central nervous system tumors, schwannomas, mesotheliomas, seminomas, teratomas and blastomas.

10. The method or the anticancer agent for use according to any one of claims 1 to 9, wherein said cancer is a non-Hodgkin lymphoma, preferably diffuse large B-cell lymphoma (DLBCL).

11. The method or the anticancer agent for use according to any one of claims 1 to 10, wherein said anticancer agent is an immunotherapeutic agent, a chemotherapeutic agent or a combination thereof.

12. The method or the anticancer agent for use according to claim 11, wherein said immunotherapeutic agent is an autologous vaccine comprising particles of hydroxyapatite and/or of tricalcium phosphate loaded with tumor antigens.

13. The method or the anticancer agent for use according to claim 12, wherein said autologous cancer vaccine comprising particles of hydroxyapatite and/or tricalcium phosphate loaded with tumor antigens, is obtained by:
a) extracting the proteins contained in a tumor, serum or plasma sample obtained from a patient suffering from cancer; and
b) bringing the proteins extracted in step a) into contact with particles of hydroxyapatite and/or of tricalcium phosphate in order to obtain the autologous vaccine.

14. The method or the anticancer agent for use according to claim 11, wherein said chemotherapeutic agent is a combination of cyclophosphamide, doxorubicin, vincristine and prednisone or prednisolone.
